# EUROPEAN PATENT SPECIFICATION

(11) **EP 0 830 111 B1**
(45) Date of publication and mention of the grant of the patent: **21.05.2003**
(21) Application number: 96918404.3
(22) Date of filing: 07.06.1996
(51) Int. Cl.: A61F 2/14, A61F 9/00

(54) **RADIAL INTRASTROMAL CORNEAL INSERT**
RADIALES INTRASTROMALES HORNHAUTIMPLANTAT
IMPLANT RADIAL DESTINE A ETRE INSERE DANS LE STROMA DE LA CORNEE

(30) Priority: 07.06.1995 US 485400
(43) Date of publication of application: 25.03.1998
(73) Proprietor: KERAVISION, INC., Fremont, California 94538-7353 (US)
(72) Inventor: SCHANZLIN, David, J., St. Louis, MO 63124 (US); VERITY, Steven, M., St. Louis, MO 63144 (US); SILVESTRINI, Thomas, A., Alamo, CA 94507 (US); PROUDFOOT, Robert, A., Santa Clara, CA 95057 (US)
(74) Representative: Price, Nigel John King
(86) International application number: US9609768
(87) International publication number: WO96040005

(56) References cited:
- WO-A-92/00112
- WO-A-94/03129
- WO-A-95/03755
- US-A- 5 098 443
- DATABASE WPI Section PQ, Week 8740 Derwent Publications Ltd., London, GB; Class P, AN 87-283436 XP002014787 & SU,A,1 296 153 (EYE DISEASE RES. INS.) , 15 March 1987
- DATABASE WPI Section PQ, Derwent Publications Ltd., London, GB; Class P, AN 84-229697 XP002014788 & SU,A,1 066 591 (TARTU. UNIV.)

## Description

### Field of the Invention

This invention is an intrastromal corneal insert designed to be placed into an interlamellar pocket or channel made within the cornea of a mammalian eye. The insert has a shape which, when inserted into the cornea, has a significant radial or meridional dimension and may be used to adjust comeal curvature and thereby correct or improve vision abnormalities such as hyperopia. The inserts may also have a circumferential component to their configuration to allow concurrent correction of other vision abnormalities. The radial insert may be made of a physiologically compatible material, e.g., one or more synthetic or natural, soft, firm, or gelatinous polymers. In addition, the insert or segment may be used to deliver therapeutic or diagnostic agents to the corneal interior or to the interior of the eye.

One or more of the radial inserts of this invention typically are inserted into the cornea so that each subtends a portion of the meridian of the cornea outside of the cornea's central area, e.g., the area through which vision is achieved, but within the cornea's frontal diameter. Typically, the insert is used in arrays of two or more to correct specific visual abnormalities, but may be used in isolation when such is called for.

### Background of the Invention

Anomalies in the overall shape of the eye can cause visual disorders. Hyperopia ("farsightedness") occurs when the front-to-back distance in the eyeball is too short In such a case, parallel rays originating greater than 20 feet from the eye focus behind the retina. Although minor amounts of hyperopia can be resolved in the human eye by a muscular action known as "accommodation", aging often compromises the ability of the eye adequately to accommodate. In contrast, when the front-to-back distance of eyeball is too long, myopia ("nearsightedness") occurs and the focus of parallel rays entering the eye occurs in front of the retina. Astigmatism is a condition which occurs when the parallel rays of light do not focus to a single point within the eye, but rather have a variable focus due to the fact that the cornea refracts light in a different meridian at different distances. Some degree of astigmatism is normal, but where it is pronounced, the astigmatism must be corrected.

Hyperopia, myopia, and astigmatism are usually corrected by glasses or contact lenses. Surgical methods for the correction of such disorders are known. Such methods include radial keratotomy (see, e.g., U.S. Patents Nos. 4,815,463 and 4,688,570) and laser corneal ablation (see, e.g., U.S. Patent No. 4,941,093).

Another method for correcting those disorders is through the implantation of polymeric rings (intrastromal corneal rings or "ICR's") in the eye's corneal stroma to change the curvature of the cornea. Previous work involving the implantation of polymethylmethacrylate (PMMA) rings, allograft corneal tissue, and hydrogels is well documented. One of the ring devices involves a split ring design which is inserted into a channel previously dissected in the stromal layer of the cornea. A minimally invasive incision is used both for producing the channel and for inserting the implant. See, for instance, the use of PMMA intrastromal rings in U.S. Patents Nos. 4,452,235 to Reynolds; 4,671,276 to Reynolds; 4,766,895 to Reynolds; and 4,961,744 to Kilmer et al. These documents suggest only the use of ICR's which completely encircle the cornea.

The use of soft polymers as intrastromal inserts is not widely known. For instance, U.S. Patent Nos. 5,090,955 and 5,372,580, to Simon, suggest an ICR which is made by introducing a settable polymer or gel into an intrastromal channel which has been previously made and allowing the polymer to set. This procedure does not allow the surgeon to specify the precise size of the resulting ring nor is it a process which allows precise control of the pathway of the flowing polymer within the eye since the gel must simply conform to the shape of the intrastmmat channel. However, it does show the concept of using arcuate channels containing a gel-based insert centered on the cornea.

Temirov et al., "Retractive circular tunnel keroplasty in the correction of high myopia", Vestnik Oftalmologii 1991: 3-21-31, suggests the use of collagen thread as ICR material.

The publications do not imply that the devices may be used to introduce therapeutic or diagnostic materials into the corneal intrastromal space.

International patent publication WO95/03755 discloses a preformed intrastromal circumferential corneal insert, made of physiologically compatible polymer, for use in adjusting corneal curvature to correct for vision abnomalities as myopia and/or astigmatism.

These publications do not suggest the introduction of polymeric inserts having significant radial or meridional dimensions into the cornea for the correction of various visual aberrations.

### Summary of the Invention

This invention is a polymeric insert suitable for insertion between the lamella of the corneal stroma according to claim 1 or claim 12. The insert may be of any of a variety of shapes, including straight, lozenge-shaped, arcuate, cross-shaped, anchor-shaped, button-shaped or but in any event has a significant radial or meridional component when inserted into the cornea. The insert may be used in isolation, in arrays of isolated multiple inserts, in cooperative multiples, as segments in a larger assemblage encircling at least a portion of the cornea, or as assemblages to form constructs of varying thickness.

The insert may be of one or more synthetic or natural polymers, hydrophilic or hydrophobic, or may be a hybrid device comprising layered materials. Optionally, the insert may contain filamentary material in the form of a single or multiple threads, random-included filaments, or woven mattes to reinforce the insert during, e.g., insertion or removal from the intrastromal channel.

The insert may be hollow and may be filled with a biologic agent, drug or other liquid, emulsified, or time-release eye treatment or diagnostic material. The insert may contain a gel, viscous, or visco-elastic material which remains in such a state after introduction. The insert may be a gel. The insert may be an injectable solid which deforms upon introduction but conforms to the form of the previously formed injection site in the cornea upon relaxation at the chosen site.

When a hybrid, the inner portion may comprise variously a composite of low modulus polymers or a single low modulus polymer. The inner portion may also comprise a polymeric material which is polymerized *in situ* after introduction into the hollow center layer.

These inventive segmented inserts may be introduced into the corneal stroma using techniques involving the steps of providing an intrastromal pocket or channel. The intrastromal pocket into which the insert is placed is, in its most simple variation, a pocket having an opening somewhere in its length into which the insert is placed. The pocket typically will have its outer end near the outer periphery of the cornea and proceeds from there towards the center of the cornea but stopping short of the sight area of the cornea. If the insert has a circumferential component as well, the pocket may be modified to include a channel which traverses at least a portion of the circumcorneal rotation to accommodate that circumferential dimension.

Specific indications, such as astigmatism, may also be rectified by insertion of one or more of the inserts into a partial intrastromal channel to steepen the center of the corneal surface. The inserts need not be of the same size, thickness, or configuration.

If hydratable polymers are used, they may be hydrated before or after introduction into the intrastromal pockets or channels created by the surgical device used to introduce these devices into the eye. If the outer layer is hydrated before insertion into the eye, the final size of the insert may be set before that insertion. If the hydratable polymers are allowed to hydrate within the corneal space, the device (if appropriate polymers are chosen) will swell within the eye to its final size. If prehydrated, the outer layer often provides a measure of lubricity to the device, allowing it to be inserted with greater ease. Other of the noted low modulus polymers may also provide such lubricity.

### Brief Description of the Drawings

Figure 1 is a schematic illustration of a horizontal section of the eye.
Figure 2 is a schematic illustration of the anterior portion of the eye showing the various layers of the cornea.
Figures 3A and 3B show respectively a front view and a cross section of a typical array of two intracorneal inserts made according to the invention.
Figures4A, 4B, and 4C show typical inserts made according to the invention explaining the conventions and terms used in explaining this invention.

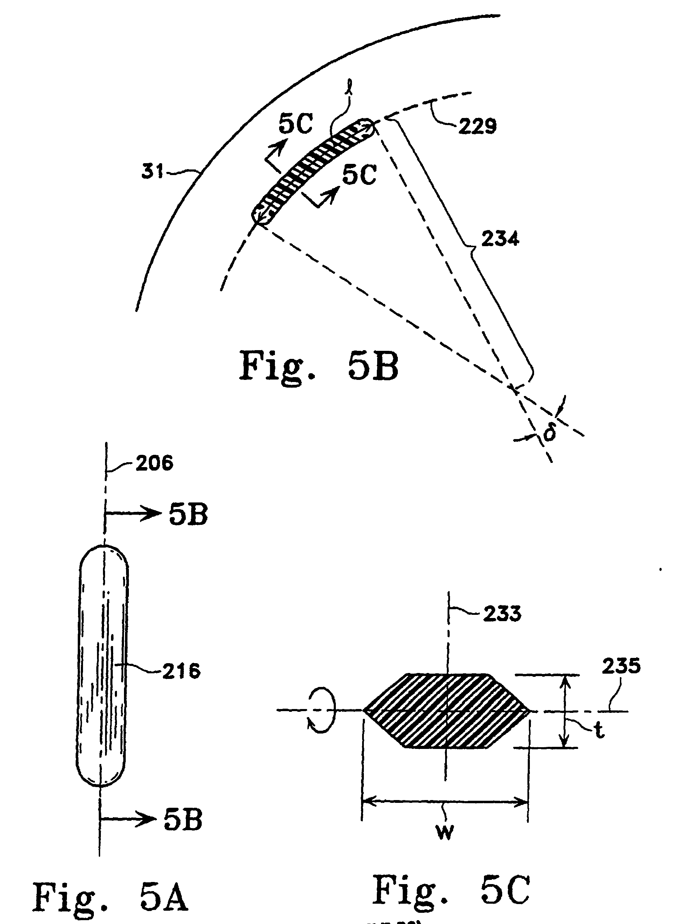

Each of Figures 5A and 5B, 6A and 6B, 7A and 7B, and 8A and 8B show respectively a front view ("A" drawing) and a side view ("B" drawing) of various intracorneal inserts made according to the invention. Figures SC and 6C show cross section of the 5B and 6B inserts, respectively.

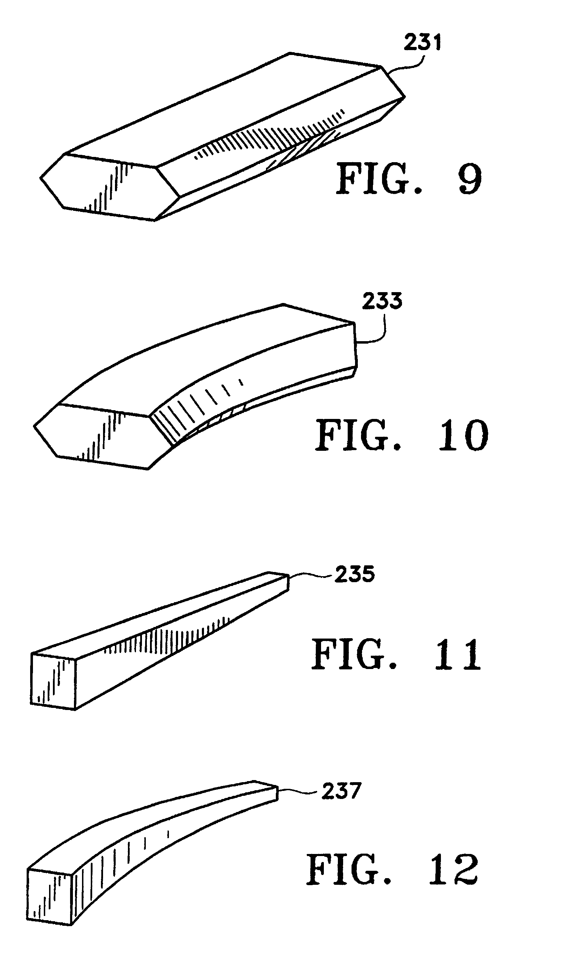

Each of Figures 9 to 15 show perspective views of variations of the inventive insert.

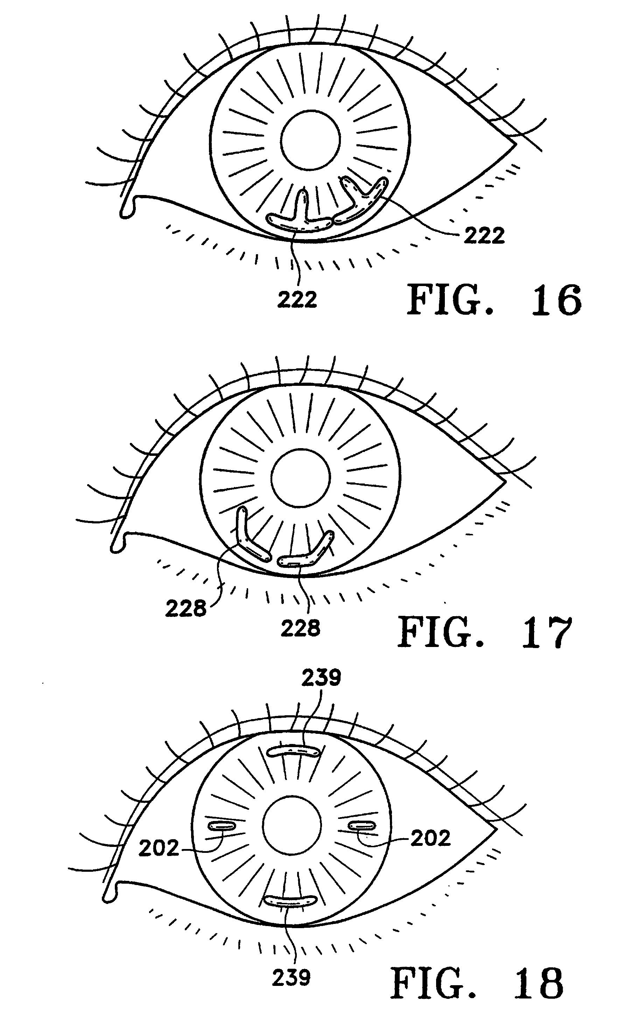

Figures 16 and 17 show respectively a front view of two inventive intracorneal inserts placed in circumferential contact with each other.

Figure 18 shows a front view of a eye having both radial inserts and circumferential segments placed therein.

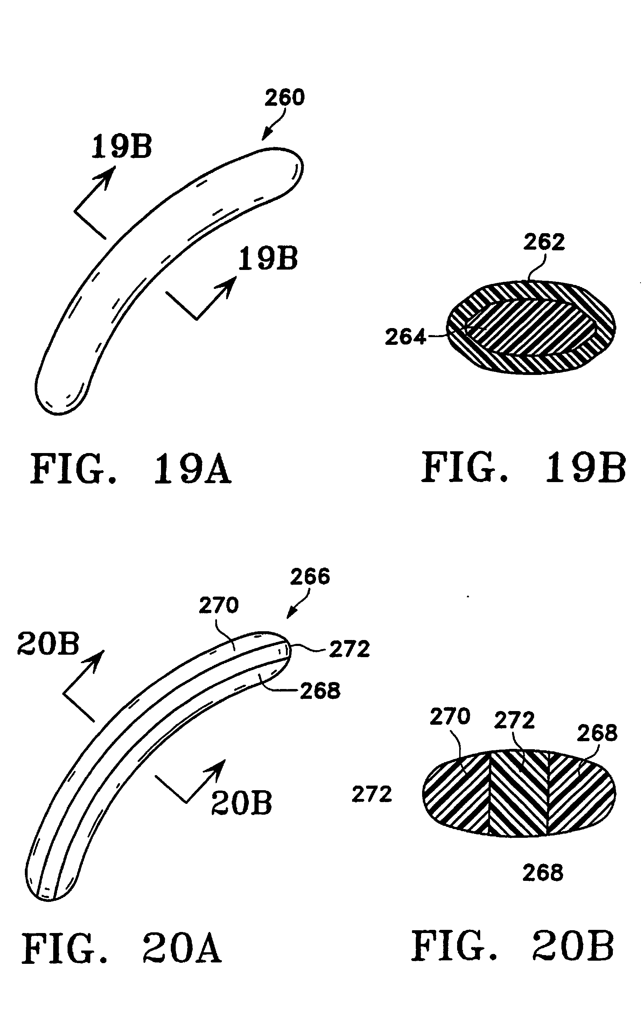

Figures 19A and 19B show respectively a front view and a cross section of a soft, filled intracorneal insert made according to the invention.

Figures 20A and 20B show respectively a front view and a cross section of a layered, composite intracomeal insert made according to the invention.

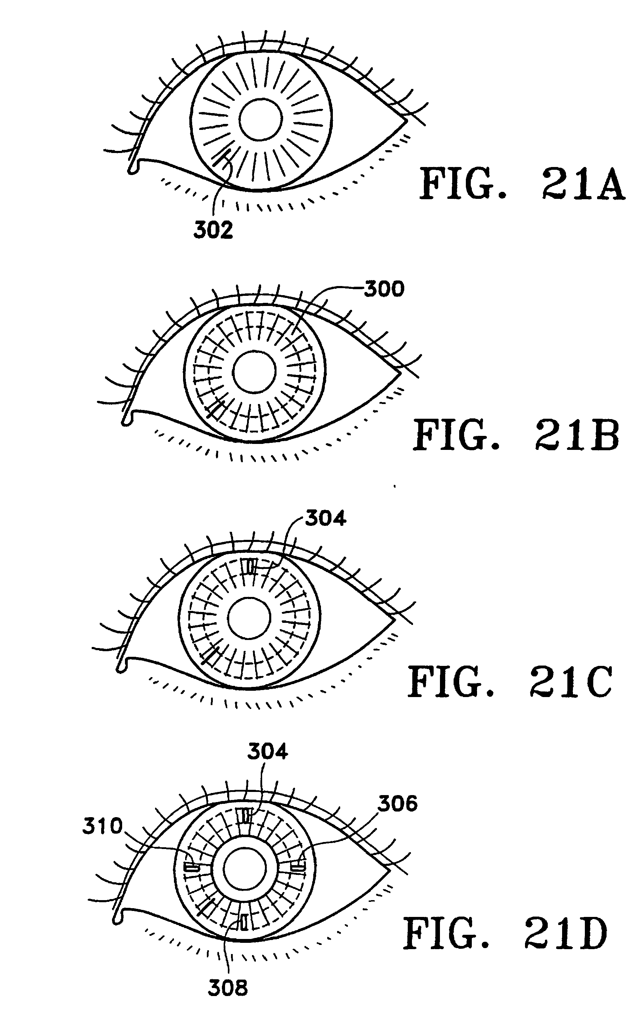

Figures 21 to 24 schematically depict procedures for introducing the inventive inserts into the cornea.

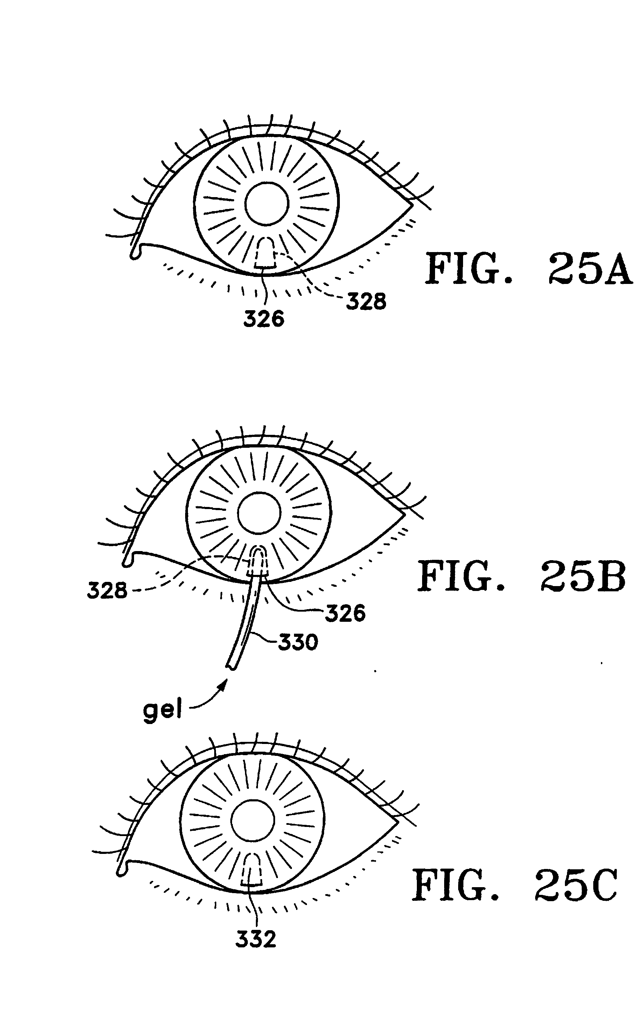

Figure 25 shows a procedure for introducing a gel into meridional pockets in the cornea.

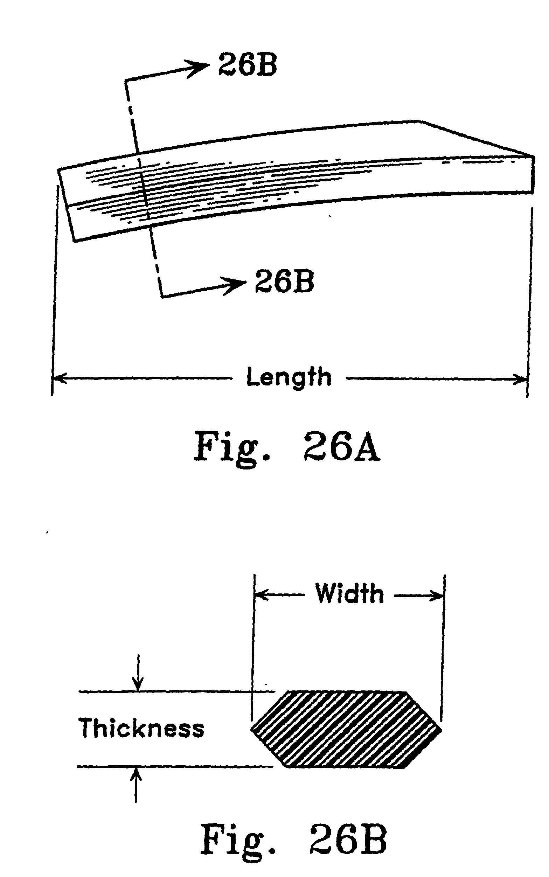

Figures 26A and 26B show respectively a side view and a cross section of an insert having a partially tapered end made according to the invention.

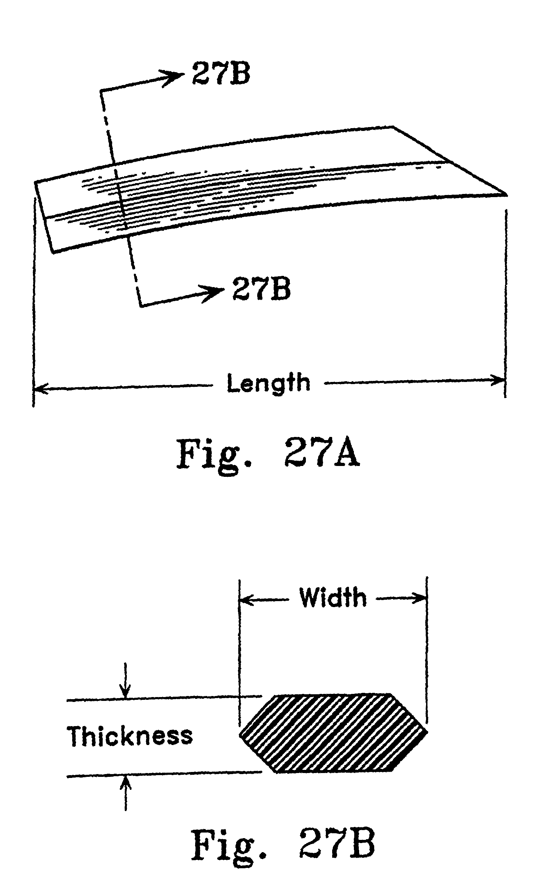

Figures 27A and 27B show respectively a side view and a cross section of an insert having a fully tapered end made according to the invention.

### Description of the Invention

Prior to explaining the details of the inventive devices, a short explanation of the physiology of the eye is needed to appreciate the functional relationship of these intracorneal inserts or segments to the eye.

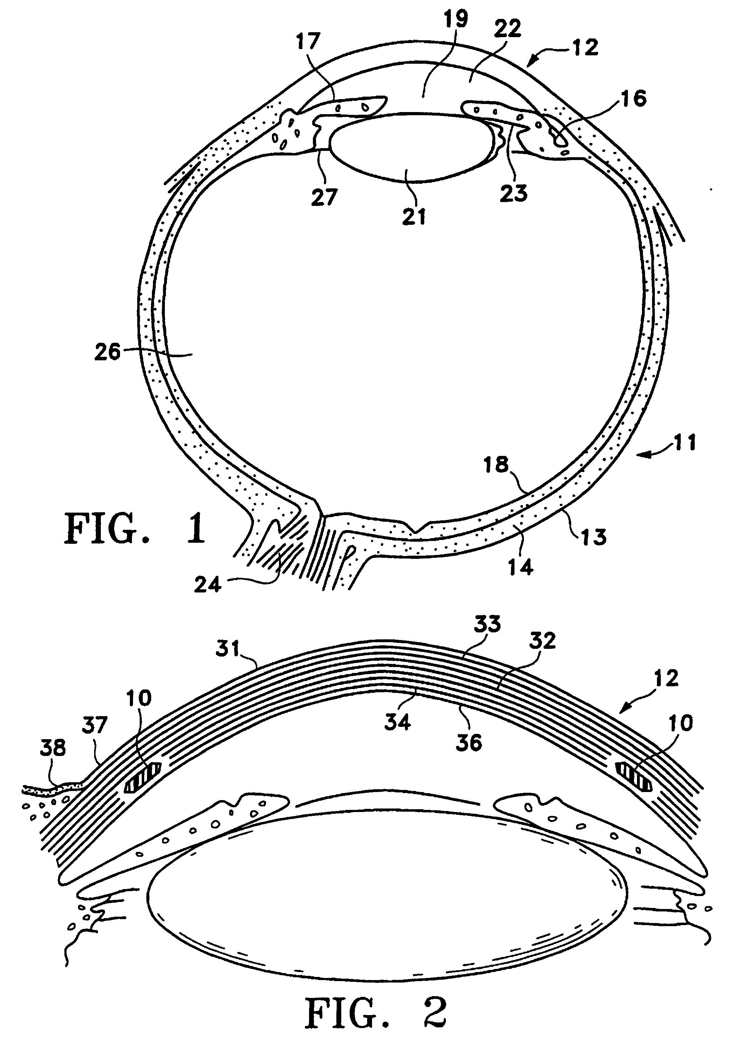

Figure 1 shows a horizontal cross-section of the eye with the globe (11) of the eye resembling a sphere with an anterior bulged spherical portion representing the cornea (12).

The globe (11) of the eye consists of three concentric coverings enclosing the various transparent media through which the light must pass before reaching the light-sensitive retina (18). The outermost covering is a fibrous protective portion the posterior five-sixths of which is white and opaque and called the sclera (13), and sometimes referred to as the white of the eye where visible to the front. The anterior one-sixth of this outer layer is the transparent cornea (12).

A middle covering is mainly vascular and nutritive in function and is made up of the choroid, ciliary body (16), and iris (17). The choroid generally functions to maintain the retina (18). The ciliary body (16) is involved in suspending the lens (21) and accommodation of the lens. The iris (17) is the most anterior portion of the middle covering of the eye and is arranged in a frontal plane. It is a thin circular disc similar in function to the diaphragm of a camera, and is perforate near its center by a circular aperture called the pupil (19). The size of the pupil varies to regulate the amount of light which reaches the retina (18). It contracts also to accommodation, which serves to sharpen the focus by diminishing spherical aberration. The iris divides the space between the cornea (12) and the lens (21) into an anterior chamber (22) and the posterior chamber (23). The innermost portion of covering is the retina (18), consisting of nerve elements which form the true receptive portion for visual impressions.

The retina (18) is a part of the brain arising as an outgrowth from the fore-brain, with the optic nerve (24) serving as a fiber tract connecting the retina part of the brain with the fore-brain. A layer of rods and cones, lying just beneath a pigmented epithelium on the anterior wall of the retina serve as visual cells or photoreceptors which transform physical energy (light) into nerve impulses.

The vitreous body (26) is a transparent gelatinous mass which fills the posterior four-fifths of the globe (11). At its sides it supports the ciliary body (16) and the retina (18). A frontal saucer-shaped depression houses the lens.

The lens (21) of the eye is a transparent bi-convex body of crystalline appearance placed between the iris (17) and vitreous body (26). Its axial diameter varies markedly with accommodation. A ciliary zonule (27), consisting of transparent fibers passing between the ciliary body (16) and lens (21) serves to hold the lens (21) in position and enables the ciliary muscle to act on it.

Referring again to the cornea (12), this outermost fibrous transparent coating resembles a watch glass. Its curvature is somewhat greater than the rest of the globe and is ideally spherical in nature. However, often it is more curved in one meridian than another giving rise to astigmatism. A central third of the cornea is called the optical zone with a slight flattening taking place outwardly thereof as the cornea thickens towards its periphery. Most of the refraction of the eye takes place through the cornea.

Figure 2 is a more detailed drawing of the anterior portion of the globe showing the various layers of the cornea (12) making up the epithelium (31). Epithelial cells on the surface thereof function to maintain transparency of the cornea (12). These epithelial cells are rich in glycogen, enzymes and acetylcholine and their activity regulates the corneal corpuscles and controls the transport of water and electrolytes through the lamellae of the stroma (32) of the cornea (12).

An anterior limiting lamella (33), referred to as Bowman's membrane or layer, is positioned between the epithelium (31) and the stroma (32) of the cornea. The corneal stroma (32) are made up of lamellae having bands of fibrils parallel to each other and crossing the whole of the cornea. While most of the fibrous bands are parallel to the surface, some are oblique, especially anteriorly. A posterior limiting lamella (34) is referred to as Descemet's membrane. It is a strong membrane sharply defined from the stroma (32) and resistant to pathological processes of the cornea. The endothelium (36) is the most posterior layer of the cornea and consists of a single layer of cells. The limbus (37) is the transition zone between the conjunctiva (38) and sclera on the one hand and the cornea (12) on the other.

With that background in place, our invention centers on the finding that introduction of an insert into the cornea, typically and desirably between the lamellar layers making up the cornea, in a position meridional to the cornea results in an alleviation of hyperopia. Although we do not wish to be bound by theory, we believe that the introduction of these radial segments results in a steepening of the center of the cornea. There may be other beneficial effects to a specific corneal surface, e.g., correction of myopia and astigmatism, but the correction to hyperopia is apparent. The meridional component to the device for correction of hyperopia may also be combined with the effects we have earlier found relating to the introduction of inserts (of this and other designs) circumferentially around the periphery of the cornea to alleviate myopia and similar problems and inserts of varying thickness at the periphery to alleviate astigmatism. The inserts may be teamed with other intrastromal corneal rings (ICR's) or partial rings or segments which are placed circumferentially about the periphery of the cornea so to correct independently a number of different ocular irregularities.

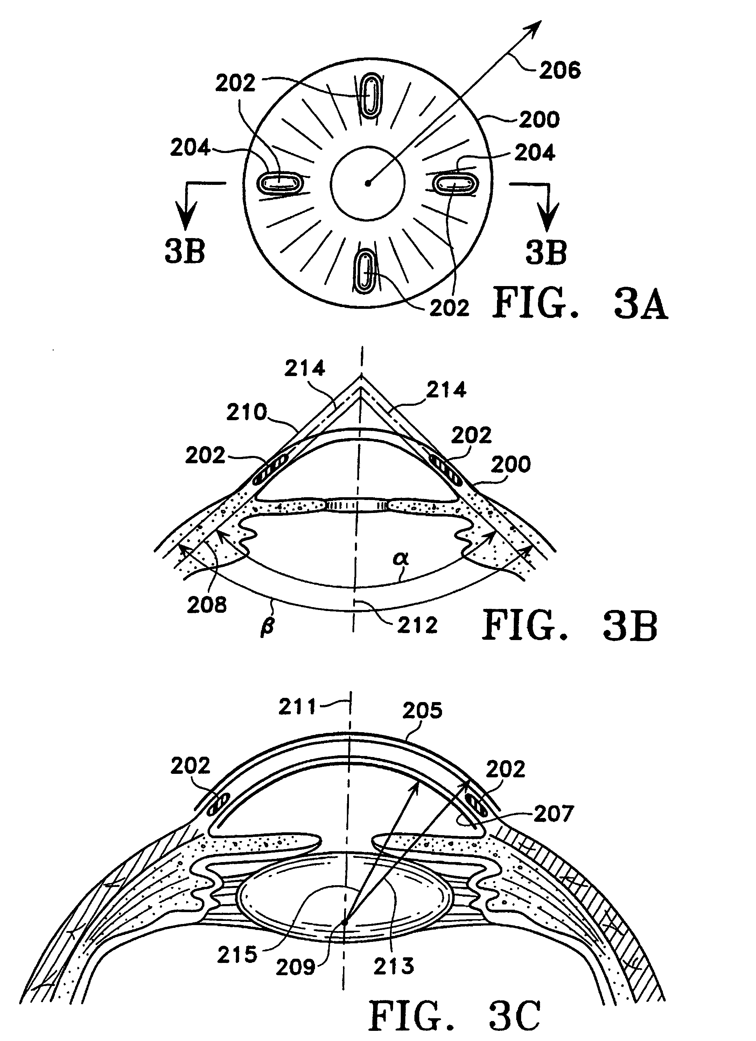

Figure 3A is a frontal view of the cornea (200) of an eye having four or an "array" of inserts (202) which are located within small meridional pockets (204). The inserts (202) are placed generally on a meridian (206) of the cornea. By "meridian" we mean the typical meaning: the direction of a line beginning at the center of the cornea, as viewed from the front of the eye, and extending outwardly towards the outer circumference of the cornea.

In any case, it is this meridional placement and sizing which is believed to cause the steepening of the center of the cornea (200) as discussed above.

Figure 3B shows a side view cross section of the cornea (200) of Figure 3A and has imposed upon it a pair of conic surfaces (208 & 210) sharing a common conic axis (212) and a common conic direction in that the apex of conic surface (208) is within conic surface (210). The volume between the two conic surfaces (208 & 210) forms a mathematical solid in which the inserts (202) lie. The length of the inserts (202), if extended as shown with a dashed line (214), extends toward the common conic axis (212). The cone angles of the two conic surfaces (208 & 210), respectively α and β, typically are independently between 15° and 60°. Although the two conic surfaces are (208 & 210) shown to be approximately parallel, this invention also includes the variation in which the two conic surfaces converge beyond the periphery of the cornea (200).

Figure 3C shows another explanation of the relationship of the various radial inserts to each other within the cornea. As was the case with the relationship shown in Figure 3B, one or more inserts (202) may be placed within the cornea as shown frontally in Figure 3A. Figure 3C shows the two inserts (202) visible in this side view. Other inserts may also be present in the cornea which are not visible in this view. The inserts are situated with respect to each other in the following manner: two or more such inserts are found between two partial hemispheres or spherical shells (205 and 207) which generally share a common center (209) or center-line (211). The radius (213) of the larger partial hemisphere (205) results in a surface approximating the anterior surface of the cornea. The radius (215) of the smaller partial hemisphere (207) results in a surface approximating the posterior surface of the cornea. The typical length of the larger radius (213) for most human eyes is between 6.7 mm and 9.0 mm. The typical length of the smaller radius (215) for most human eyes is between 5.5 mm and 7.0 mm. The typical thickness of a human cornea (and hence the difference between the length of larger radius (213) and smaller radius (215)) is between 0.4 mm and 0.8 mm. The inserts (204) are situated in the same generally meridional position as depicted in Figure 3A -- that is to say that the insert (204) desirably lies in a position which, if extended, would intersect the centerline (211).

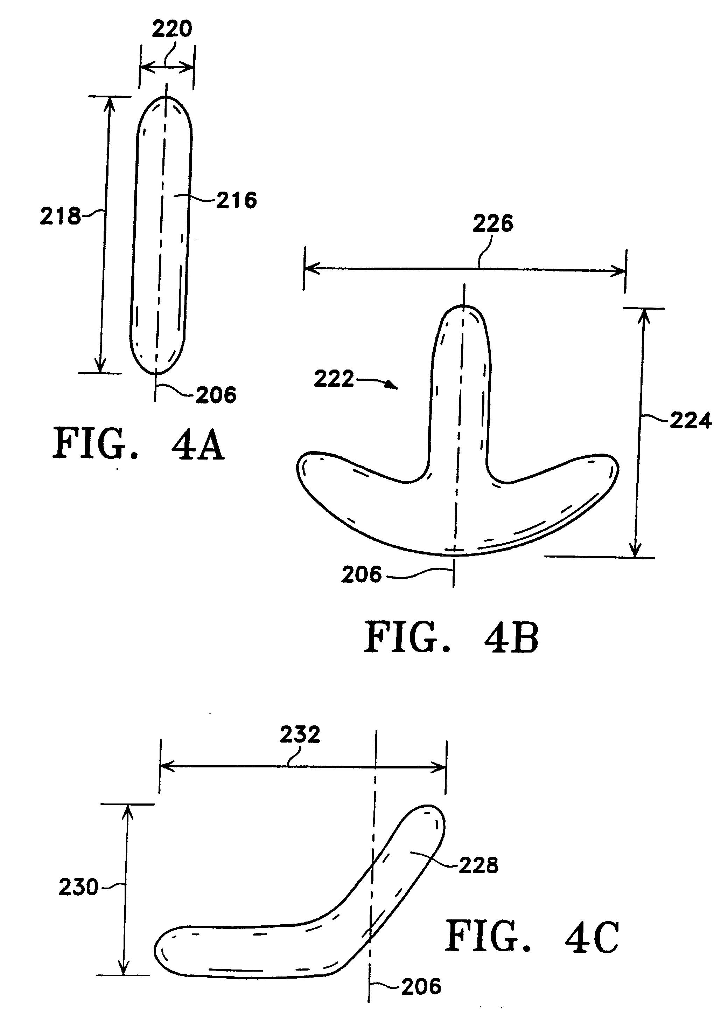

Figures 4A, 4B, and 4C show front views of variations of the inventive inserts, provide explanations of the conventions used in defining the meridional and circumferential dimensions, and show general orientation of the inserts in the cornea. For instance, in Figure 4A is seen one of the simplest forms of inserts made according to the invention. There, the insert (216) has a significant meridional length component (218) lying approximately along comeal meridian (206), and only a small width or circumferential component (220). The ratio of the length of the meridional length component (218) to the width or circumferential component (220) is typically greater than 1.0, preferably 1.5 to 20.0 and, for the simple insert (216) shown in Figure 4A, may even be 20.0 or more.

Figure 4B shows an insert (222) having both a significant meridional length component (224) and a significant meridional length component (224) and a significant width or circumferential component (226). The ratio of the length of the meridional length component (224) to the width or circumferential component (226) in the variation shown in Figure 4B is about 1.0. Its general positioning to the corneal meridian (206) is also depicted in the Figure.

Figure 4C also shows an insert (228) having both a significant meridional length component (230) and a significant width or circumferential component (232). The ratio of the length of the meridional length component (230) to the width or circumferential component (232) in the variation shown in Figure 4C is also about 1.0.

The concept of measuring the meridional length of the inserts by observing the length of the insert which falls along a meridian (206) of the cornea should be clear from the examples shown in Figures 4A, 4B, and 4C.

Figure 5A shows a front view of an insert (216) made according to the invention. Figure 5B shows a side view of the Figure 5A insert in relation to the anterior corneal surface which follows the external epithelium (31). The side view shows a desirable embodiment in which the insert's centroidal axis follows an intracorneal arc (229) in a direction parallel to a comeal meridian, and if not pliable, exhibits a pre-shaped radius of curvature (234) before implantation, as well as after implantation. Such a device is referred to as "radially arcuate." The radius of curvature (234) approximates, *e.g*., lies between, the hemispherical radii (213 and 215) shown in Figure 3C at the depth of implantation in the cornea into which it is placed. This radius of curvature (*i*.*e*., the radius of curvature measured along the centroidal axis of the insert, or that portion of the insert intended to extend generally radially within the cornea) is preferably greater than 5 mm, more preferably greater than 5.5 mm, and typically ranges from 6 to 9 mm. In a preferred embodiment, the radius of curvature ranges from 7 to 8 mm.

Figure 5B also shows that the insert has a centroidal length ("ℓ") measured along its centroidal axis at a given radius of curvature (234). This centroidal length "ℓ" subtends an arc having an angle "δ". This value is referred to herein as the "meridional arc angle". The value of δ is preferably less than or equal to 90°, and more preferably less than or equal to 45°.

Figure 5C shows a cross sectional view of the Figure 5B device. As shown, the hexagonal cross section of the insert gives rise to two axes; a short axis (233) which lies in the same plane as the meridional component and a long axis (235) perpendicular to this plane. The short axis defines the thickness of the insert, "t" whereas the long axis defines the width of the insert, "w". As depicted in Figures 5B and 5C, the radially arcuate insert subtends a meridional arc around an axis which is generally parallel to the long axis. Variations of the insert in which pliable polymers or gels are used need not be pre-formed in this way. The variation of Figures 5A, 5B and SC desirably tapers to a blunt point at one or both of the ends of the device. Such a configuration allows for ease of insertion and reduces trauma to eye tissue.

The concept of measuring the centroidal length of the insert by observing the length of the insert along the centroidal axis of the insert which extends in the direction of a corneal meridian (206), should be clear from the examples shown in Figures 5A, 5B, and 5C.

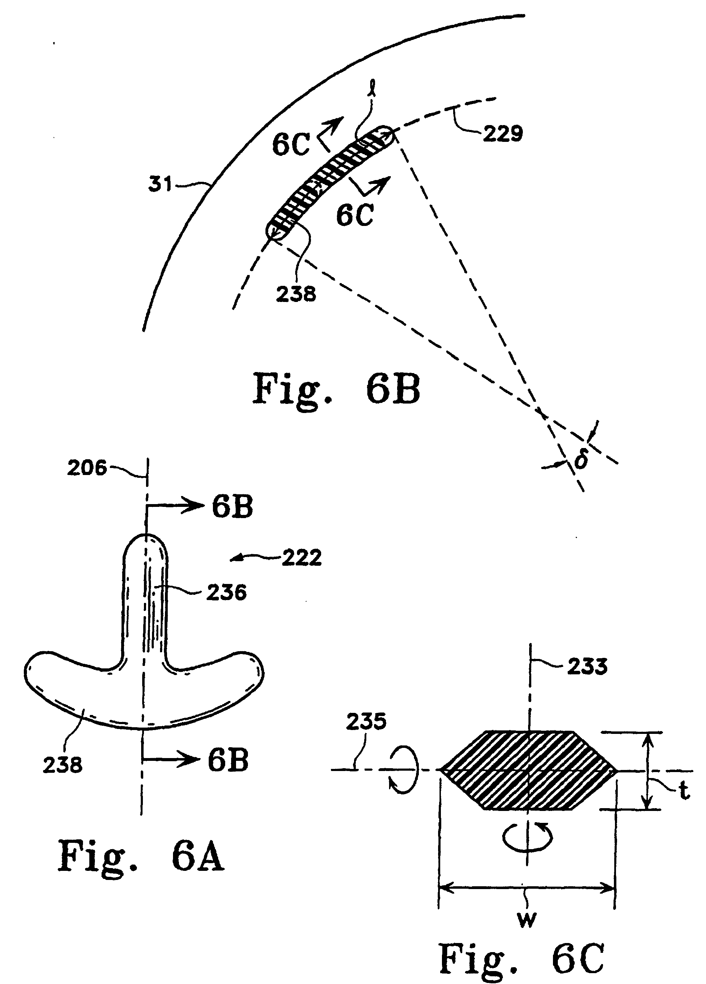

Figure 6A shows an anchor-shaped variation (222) of the inventive insert having a radial leg (236) and a circumferential portion (238). Such an insert is referred to as a "combination insert." Although this variation has a significant radial leg (236), the correction of hyperopia may be secondary in importance. The circumferential portion (238) subtends a certain portion of the circumference of the cornea along an arcuate path around (*i.e*., perpendicular to) the short axis (235), and is thus said to be "circumferentially arcuate", and may accordingly effect the correction of other maladies, e.g., keratoconus. In addition, the insert may subtend an arcuate path in a third dimension around an axis which corresponds to the meridional radius. There is some interaction between the portions of the combination insert, but generally the thickness, length, and width of the sections may be varied to independently correct the noted vision acuity maladies.

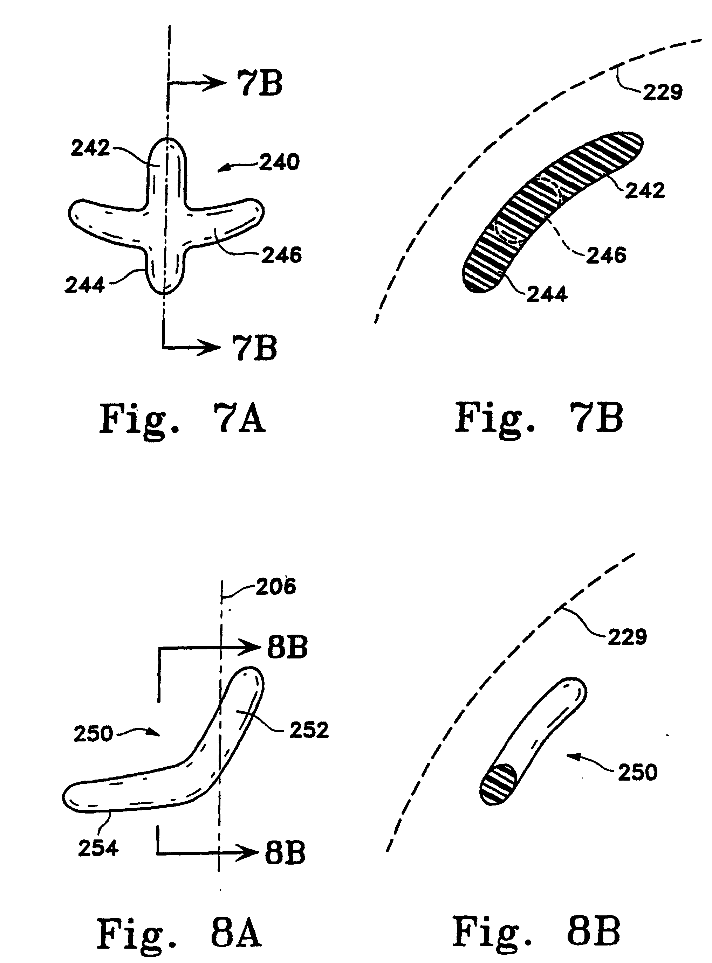

Figure 7A shows a front view of a cruciform-shaped variation (240) of the inventive insert having an inner radial leg (242) and a outer radial leg (244) as well as a circumferential portion (246). Again, the side view found in Figure 7B shows an optional corneal radius of curvature such as discussed in relation to Figures 5A and 5B.

Figure 8A shows a front view of a boomerang-shaped variation (250) of the inventive insert having a radial leg (252) and a circumferential portion (254). The side view found in Figure 7B shows an optional radius of curvature. The radial leg (252) is not situated in such a way that it is placed in line with a meridian of the cornea but it can be conceptualized as being a distributed or functionally wider radial leg.

Further, the typical width of the individual inserts discussed above is often between 0.2 mm and 2.0 mm. The typical thickness is often between 0.15 mm and 0.5 mm. In addition to the width and thickness of the insert tapering at one or both ends, the thickness of the insert may optionally vary from one end to the other end of the insert (*e*.*g*., along the centroidal length of the insert) to provide for a desired change in corneal curvature at the location of the insert. The centroidal length of the insert (*i*.*e*., the length of the insert measured along the centroidal axis of the insert) is contemplated to rarely exceeds 3.0 mm. Preferably, the insert has a centroidal length which is less than or equal to 2.5 mm, and more preferably less than 2.0 mm. When the centroidal length is determined for an insert configuration other than the simple configuration shown in Figure 4A (*e.g*., such as the insert shown in Figures 4B), the centroidal length corresponds to the length of the radially arcuate portion measure along the centroidal axis of that portion. As another example (*e.g*., the insert of Figure 4C), this length corresponds to the length of the generally radially extending leg (*e.g*., the non-circumferentially extending portion) measured along its centroidal axis. These parameters (along with certain other variables such as the cross-sectional shape of the device and its constituent polymers and stiffness) determine, in large part, the level of correction achievable by use of a selected insert.

Other non-limiting forms for the inventive insert are exemplified in Figures 9-15. Figure 9 shows a perspective view of an insert (231) having a generally hexagonal cross section. The insert (239) is shown to be straight, that is, not to have a shape which conforms to the curvature of the anterior corneal surface prior to its introduction into the eye. Consequently, this variation would likely be produced of a material which is pliable and able to conform to a pocket previously formed in the cornea.

Figure 10 shows a further variation (233) of the device in Figure 9. This device (233) has a pre-form curve to it and, consequently, the inventive insert may be made of a stiff or pliable material.

Figure 11 shows a square cross-section variation (235) of the device which is straight when not confined in the corneal channel. A gentle taper of the device (235) may be seen in the Figure. Either end of the device (25) and others described herein having tapered shapes, may be inserted into the eye using either the thin or fat end extending toward the outer periphery of the cornea. Typically, however, the fatter end will be placed at the outer periphery. The rate of taper from one end of the device is not important to this invention and need not be linear along the axis of the device, but may be of any convenient form.

Figure 12 shows a further variation (237) of the device (235) shown in Figure 11. In this instance, the device (237) is curved or has "preform" as well as having an axially variable cross-section.

The variations of this invention actually depicted in the drawings are considered to be only examples of the wide range of specific devices suitable for use in this inventive concept.

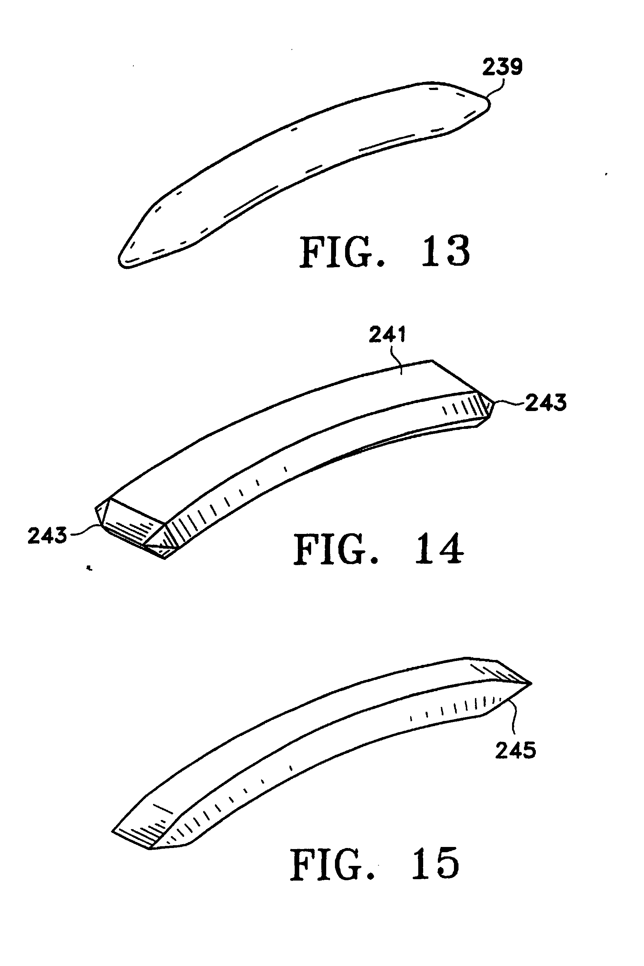

Figure 13 shows a variation (239) of the invention similar in shape to the device shown in Figures 5A and 5B. This variation (239) and the others shown in Figures 14 and 15 have tapered ends to ease introduction of the device into the eye and lessen the trauma it may cause during that introduction and during use of the device. The insert (239) shown in Figure 13 has generally smooth anterior and posterior surfaces and somewhat blunt opposing ends. It (239) is shown to be curved although such a form is not required.

Figure 14 shows a variation of the devices shown in Figures 9 and 10 in which one or more of the ends (243) have a tapered or blunt shape.

Similarly, Figure 15 shows a variation of the devices (245) shown in Figures 11 and 12 in which one or more of the ends have a tapered or blunt shape.

It should be apparent that these devices may be sterilized using known procedures having sterilants such as ethylene oxide or radiation (if the chosen materials so permit). The devices must be sterilized prior to use. It would be a normal practice to package these devices in ways using packages known for other ophthalmic devices capable of preserving the sterilization state. A typical commercial packaged, sterilized device would contain at least one device in such a sterile package. Depending upon the chosen materials for the insert, the packaging might be dry and include an inert gas or might contain a sterile fluid such as saline solution.

Figures 16 and 17 show variations of the invention in which multiple inserts are included in an intralamellar tunnel included within a human eye. Figure 16 is intended to demonstrate a variation in which a number of inserts (222) are placed contiguously in an array within the channel rather than in an equally spaced array as was described in conjunction with Figure 3A.

Figure 17 shows a variation in which two inventive inserts (228) are nearly contiguous within the intrastromal channel.

Figure 18 shows an array of two inventive radial inserts (202) in conjunction with two circumferentially placed intrastromal segments (239). The radial inserts (202) are placed in the cornea for the purposes noted above, typically hyperopia correction and perhaps astigmatism correction, and the circumferential segments (239) are introduced for myopia or astigmatism correction. A complete disclosure of the structure and use of the segments (239) may be found in WO95/03755, entitled SEGMENTED PREFORMED INTRASTROMAL CORNEAL INSERT, filed August 2, 1993 and 08/101,440, entitled SEGMENTED PLIABLE INTRASTROMAL CORNEAL INSERT, filed August 2, 1993, both by Silvestrini.

The specific array of radial inserts (202) and circumferential segments (239) is not limited to the alternating pattern shown in the Figure, nor is the invention limited to the positioning or numbers of inserts shown in the Figure. The choice of and placement of appropriate insets and segments is left to the attending health professional based upon the abnormality to be treated.

The materials used in these inserts may be relatively stiff (high modulus of elasticity), physiologically acceptable polymers such as acrylic polymers like polymethylmethacrylate (PMMA) and others; polyfluorocarbons such as TEFLON®; polycarbonates; polysulfones; epoxies; polyesters such as polyethyleneterephthalate (PET), KODAR, and Nylon; or polyolefins such as polyethylene, polypropylene, polybutylene, and their mixtures and interpolymers. Certain glasses are also suitable for the devices. By "high modulus of elasticity" is meant a modulus greater than about 24 MPa (3.5 kpsi). Many of these polymers are known in the art to be appropriately used in hard contact lenses. Obviously, any polymer which is physiologically suitable for introduction into the body is useful in the inserts of this invention. Many of the listed polymers are known to be suitable as hard contact lenses. For instance, PMMA has a long history in ophthalmological usage and consequently is quite desirable for use in these inserts.

Additionally, the polymeric material making up the insert may be one or more low modulus polymers, e.g., those having a modulus of elasticity below about 24MPa (3.5 kpsi), more preferably between 7kPa (1 psi) and 7MPa (1 kpsi), and most preferably between 7kPa (1 psi) and 3.44MPa (500 psi), which are physiologically compatible with the eye. Most polymeric materials used in soft contact lenses are suitable the inserts of this invention. The class includes physiologically compatible elastomers and such polymers, typically crosslinked, as polyhydroxyethylmethylacrylate (Poly-HEMA) or polyvinylpyrrolidone (PVP), polyethylene oxide, or polyacrylates, polyacrylic acid and its derivatives, their copolymers and interpolymers, and the like as well as biologic polymers such as crosslinked dextran, crosslinked heparin, or hyaluronic acid. Acrylic polymers having a low T_{g} are also suitable.

In many instances, the intrastromal segments may be hybrid, that is to say, the segments are made up of a number of polymeric layers typically with a soft or hydratable polymer on their outer surface. These hybrid segments will be described with greater particularity below. Partially hydrated or fully hydrated hydrophilic polymers are typically slippery and consequently may contribute to the ease with which the insert may be introduced into the interlamellar tunnel. Suitable hydrophilic polymers include polyhydroxyethylmethacylate (PHEMA), N-substituted acrylamides, polyvinylpyrrolidone (PVP), polyacrylamide, polyglycerylmethacrylate, polyethyleneoxide, polyvinyl alcohol, polyacrylic acid, polymethacrylic acid, poly (N, N-dimethyl amino propyl-N¹-acrylamide) and their copolymers and their combinations with hydrophilic and hydrophobic comonomers, crosslinks, and other modifiers. Thermoplastic hydrogels include hydropolyacrylonitrile, polyvinyl alcohol derivatives, hydrophilic polyurethanes, styrene-PVP block copolymers and the like.

The intrastromal segment may be lubricated with suitable ocular lubricants such as hyaluronic acid, methylethyl cellulose, dextran solutions, glycerine solutions, polysaccharides, or oligosaccharides upon its introduction to help with the insertion particularly if one wishes to insert intrastromal segments of hydrophilic polymers without prior hydration. If a hybrid segment having a hydrophilic polymeric covering or a segment comprising a hydrophilic polymer is inserted into the eye without prior hydration, subsequent to the insertion, the intrastromal segment will swell to its final size or thickness within the eye. This swelling often permits the inclusion of larger intrastromal segments than would normally be accommodated within normal sized intrastromal channels.

Low modulus polymers used in this invention are often absorbent, particularly if they are hydratable, and may be infused with a drug or biologic agent which may be slowly released from the device after implantation of the intrastromal segment. For instance, the low modulus polymer may be loaded with a drug such as dexamethasone to reduce acute inflammatory response to implanting the device. This drug may help to prevent undesirable vascular ingrowth toward the intrastromal segment and improve the overall cosmetic effect of the eye with the insert and segment Similarly, heparin, corticosteroids, antimitotics, antifibrotics, antiinflammatories, anti-scar-forming, anti-adhesion, and antiangiogenesis factors (such as nicotine adenine dinucleotide (NAD⁺)) may be included to reduce or prevent angiogenesis and inflammation.

Clearly, there are a variety of other drugs suitable for inclusion in the intrastromal segment. The choice will depend upon the use to which the drugs are placed.

Figure 19A is a side view of a variation of the intrastromal sector or insert (260) made of a low modulus polymer system or hydratable outer coating (262). Figure 19B shows the inner cavity (264) of the insert. This intrastromal segment may be inserted into the intrastromal space created by the dissector as a covering on a tool similar to the dissector which created the intracorneal pocket or channel. Once in position the insertion tool is rotated out of the intrastromal segment leaving the shell within the stroma.

Figure 19B shows the inner cavity (264) which may be filled with a biologic, a drug or other liquid, or biologically active eye treatment material. These devices may be tied or pinched or crimped or otherwise closed, typically at their point of insertion, by known techniques. If the inserts were closed or sealed prior to introduction, the insert may later be punctured with a syringe and a fluid or gel my be introduced or withdrawn for a variety of clinical reasons.

The shell (262) may be injected with a settable soft polymer core (264), allowed to expand to a desired thickness, and set. Polymeric gels which do not polymerize in situ are preferred. Suitable injectable polymers are well known but include polyHEMA hydrogel, cross-linked collagen, cross-linked hyaluronic acid, PVP, polyacrylonitriles, polyacrylamides, polyacrylic acids, their copolymers and terpolymers, vinyl alcohol derivatives, etc. Siloxane gels and organic-siloxane gels such as cross-linked methyl vinyl siloxane gels are acceptable but are generally considered to be less suitable.

The core (264) may also be a high or low modulus polymer if so desired.

Figure 20A shows a front view of a hybrid layered intracorneal insert (266). Viewed in cross section in Figure 20B, the multiple layers of the insert (266) may be seen. Figures 20A and 20B are intended to show the concept of a multilayered insert made up of polymers of different characteristics. In this example of a multi-layered insert, the hybrid insert has inner (268) and outer faces (270) of polymers having low moduli of elasticity.

The inner portion or core (272) may be a physiologically compatible polymer having a high modulus of elasticity or other polymer of low modulus.

If hydratable polymers are chosen for the outside layers, the extent to which those outer layers swell upon hydration is dependent upon the type of polymer chosen and, when the polymer is hydratable, upon the amount of cross-linking found in the outer layers (268) and (270), and upon the thickness of the layer. Generally speaking, the more highly linked the hydratable polymer, the smaller the amount of volume change upon hydration. Conversely, a polymer having only sufficient cross-linking for strength in the service in which this device is placed, will have a somewhat lower level of cross-linking. Alternatively, a substantially nonswellable polymer system may be formed of a hydrogel physically interpenetrated by another polymer which does not hydrate, e.g., PMMA into polyHEMA.

The thickness of the outer layer depends in large function upon the intended use of the intrastromal segment. If the outer layer is used to provide a swellable outer layer which does not add significantly to the size of the intrastromal segment or is used functionally as a lubricant layer, the other layer may be quite thin -- even to the point of a layer of minimum coverage, perhaps as thin as a single molecular layer.

Of course, the inner and outer layers need not be, respectively, low modulus and high modulus polymers but may instead be multiple layers of low modulus polymers including an outer hydrophilic polymer layer and an inner hydrophobic polymer; a variety of hydrophilic polymers; etc.

Additionally, the inventive device shown in Figures 20A and 20B need not have a inner (268) and outer (270) layers over the entire insert. For instance, an insert having a thicker portion and a substantially thinner portion may be desired. An insert having an inner core of a high modulus polymer and an outer covering of a swellable polymer might be chosen. The surgeon would remove a portion of the insert's exterior coating or face prior to introducing the insert into the eye. Further, hydrophilic polymers are more easily infused with therapeutic and diagnostic materials than are the high modulus materials. In the variation just noted, the insert may then be used to deliver the infused therapeutic and diagnostic materials in a greatly delimited treatment or diagnostic area.

Figures 21 to 24 show procedures for introducing the inventive inserts into the cornea.

Figure 21 shows a procedure for introducing the inserts into the cornea using a circumferential intrastromal channel (300). The former portion of this general procedure is described in U.S. Pat. No. 5,300,118, to Silvestrini et al, issued April 5, 1994. In step 1, a small meridional incision (302) is made in the outer periphery of the anterior surface of the cornea. The slit may be circumferential if so desired. This slit (302) does not perforate the cornea but instead terminates within the cornea itself. See, for instance, the depth of placement for the inserts shown in Figures 3B and 3C. A dissector, such as is shown in U.S. Pat. No. 5,403,335, to Loomas et al, issued April 4, 1995, is then introduced into the initial incision (302) and rotated to form the circumferential interlamellar tunnel (300) found in step 2 of Figure 21. In step 3, a first radial insert has been introduced through the initial slit (320), through the intrastromal channel (300), and rotated into a meridional position at 12:00 in the channel (300). Other inserts (306, 308, and 310) are introduced in the same fashion. The initial opening (302) is then closed by use of a suture, glue, staple, or by electrosurgical welding.

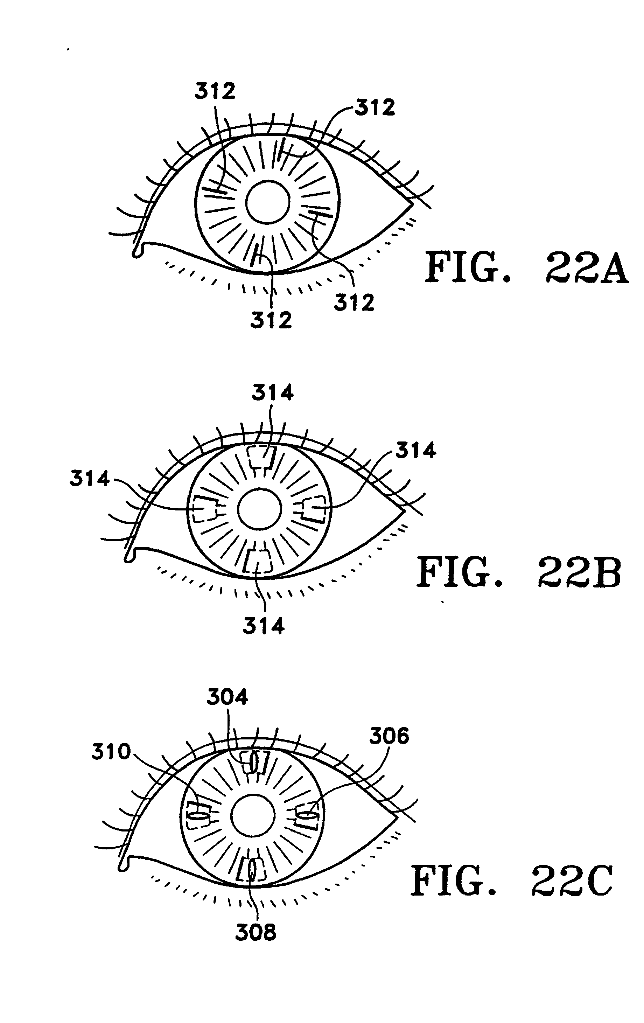

Figure 22 shows a procedure in which the various radial inserts are introduced using individual incisions. Step 1 shows the presence of four meridional incisions (312) made from the anterior surface of the cornea to a depth only partially through the cornea. Small pockets (314) are then meridionally placed from the four incisions (312) in step 2. The four inserts (304, 306, 308, and 310) are then placed at the back end of the various pockets (314) in step 3. Again, the various initial incisions (312) are then closed by use of sutures, glue, staples, or by electrosurgical welding.

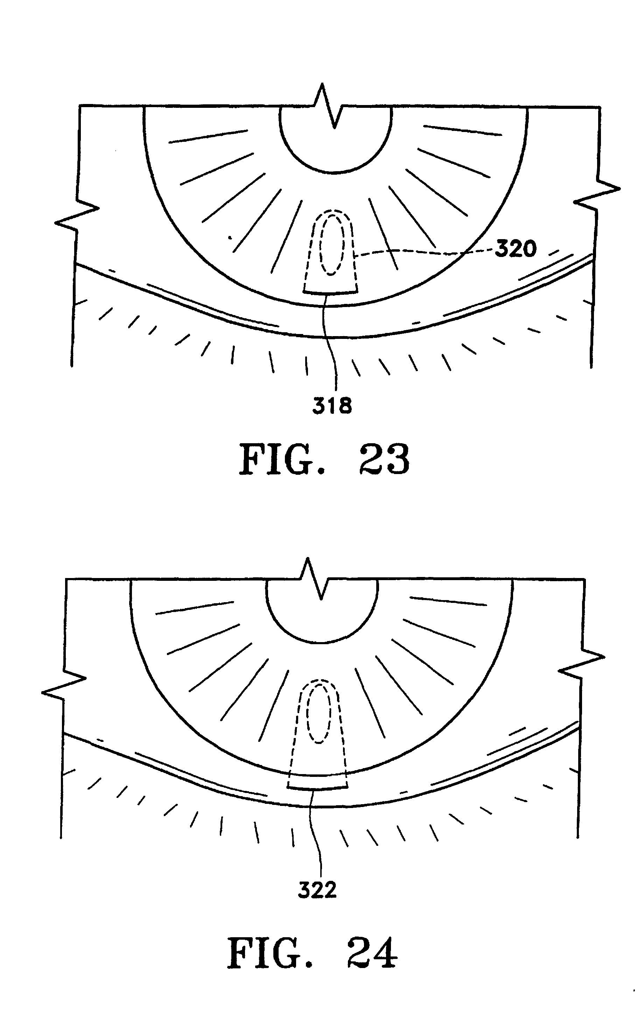

Figures 23 and 24 show entry incisions alternative to those shown in the procedure shown in Figure 22. Figure 23 shows a small circumferential incision (318) and a meridional pocket (320) for placement of the insert. Incision (318) is within the cornea.

Figure 24 shows an incision (322) outside of the cornea, within the limbus of the eye through which the insert is placed into the cornea.

In addition to the devices which make up the invention and have been described above, a method of producing radial inserts comprising only a gel may use a method similar to the surgical procedures outlined with regard to Figures 22-24. Figure 25 outlines such a procedure. In step 1, a small incision (326) is made into the cornea and a small meridional pocket (328) is formed. In step 2, a conduit (330) containing an appropriate gel is introduced into the incision (326) and the pocket (328) is filled with an appropriate amount of gel. The gel radial insert (332) is shown in position in step 3. Incision (326) has obviously been closed.

### Examples

### Example 1

Figures 26 and 27 depict the shape of various inserts which were placed intracorneally into eyes from human cadavers. Two to six inserts were placed in each eye in the direction of a corneal meridian. As shown, the Figure 26 insert is partially tapered at one end in the direction of insertion, while the Figure 27 insert is fully tapered at one end. These inserts were preformed from polymethylmethacrylate. The dimensions of the inserts were as follows:

| **Insert No.** | **Insert Shape** | **Centroidal Length (mm)** | **Width (mm)** | **Thickness (mm)** | **Radius of Curvature (mm)** |
|---|---|---|---|---|---|
| **Insert 1** | Figs. 26A and B | 2.0 | 0.80 | 0.30 | 7 |
| **Insert 2** | Figs. 26A and B | 1.5 | 0.80 | 0.30 | 8 |
| **Insert 3** | Figs. 26A and B | 2.0 | 0.80 | 0.45 | 8 |
| **Insert 4** | Figs. 27A and B | 2.0 | 0.80 | 0.30 | 7 |
| **Insert 5** | Figs. 27A and B | 1.5 | 0.80 | 0.30 | 8 |
| **Insert 6** | Figs. 27A and B | 2.0 | 0.80 | 0.45 | 8 |

The length of the inserts were measured along their centroidal axes as they extended in the general direction depicted in Figures 26A and 27A, although the centroidal axes of the inserts are not shown.

Each of the inserts changed the corneal curvature by a desired amount up to 8 diopters to provide for hyperopic correction, and exhibited stable dimensions (no appreciable changes in length, width or thickness) over a time of one hour. Devices prepared in accordance with the foregoing example which were without curvature also changed the corneal curvature by approximately the same amount.

This invention has been described and specific examples of the invention have portrayed. The use of those specifics is not intended to limit the invention in any way. Additionally, to the extent that there are variations of the invention which are within the disclosure and yet are equivalent to the inventions found in the claims, it is our intent that this patent cover those variations as well.

## Claims

1. An intracorneal insert (202, 216, 222, 240) for introduction into the cornea of a human eye, said insert comprising a physiologically compatible material and further comprising at least one elongated portion (236, 242, 244) having a component with a radius of curvature, measured along the centroidinal axis of the insert, greater than 5.0 mm, wherein the insert is adapted for implantation within a human cornea with said component extending in a meroidinal direction thereby to effect refractive correction.

2. The insert of claim 1, wherein said radius of curvature is greater than 5.5 mm.

3. The insert of claim 1, wherein said radius of curvature is between 6.0 and 9.0 mm.

4. The insert of claim 1, wherein said radius of curvature is between 7.0 and 8.0 mm.

5. The insert of claim 1, having a centroidinal length of less than 3.0 mm.

6. The insert of claim 5, wherein said centroidinal length is less than or equal to 2.5 mm.

7. The insert of claim 6, wherein said centroidinal length is less than or equal to 2.0 mm.

8. The insert of claim 1, wherein said elongated portion (236, 242, 244) extends only in a single direction.

9. The insert of claim 1, including at least two elongated portions (236, 238, 242, 244, 246) extending in different directions.

10. The insert of claim 9, wherein said insert (222) is substantially anchor-shaped.

11. The insert of claim 9, wherein said insert (240) is substantially in a cross-shaped.

12. An intracorneal refractive correction insert (222, 228, 240, 250) for introduction into the cornea of a human eye, said insert comprising a physiologically compatible material and being adapted for implantation within a human cornea, said insert further comprising a first elongated portion and a second elongated portion extending therefrom in a different direction, one of said first and said second portions being configured for radial insertion in the cornea of a human eye.

13. The insert of claim 12, wherein said first elongated portion has said second and a third elongated portion extending therefrom.

14. The insert of claim 12, wherein said insert (222) is configured in the shape of an anchor.

15. The insert of claim 12, wherein said insert (228, 250) is configured in the shape of a boomerang.

16. The insert of claim 12, wherein said insert (240) is configured in the shape of a cross.

## Patentansprüche

1. Ein intracornealer Einsatz (202, 216, 222, 240) zur Einführung in die Hornhaut eines menschlichen Auges, wobei der Einsatz ein physiologisch verträgliches Material umfaßt und weiter mindestens einen verlängerten Teil (236, 242, 244) umfaßt, welcher einen Bestandteil mit einem Krümmungsradius, gemessen entlang der zentralen Körperachse des Einsatzes, größer als 5,0 mm hat, wobei der Einsatz zur Implantation in einer menschlichen Hornhaut mit dem sich in meridianer Richtung erstreckenden Bestandteil angepaßt ist, um dadurch eine Brechungskorrektur zu bewirken.

2. Der Einsatz nach Anspruch 1, wobei der Krümmungsradius größer als 5,5 mm ist.

3. Der Einsatz nach Anspruch 1, wobei der Krümmungsradius zwischen 6,0 und 9,0 mm liegt.

4. Der Einsatz nach Anspruch 1, wobei der Krümmungsradius zwischen 7,0 und 8,0 mm liegt.

5. Der Einsatz nach Anspruch 1, welcher eine zentrale Körperlänge kleiner als 3,0 mm hat.

6. Der Einsatz nach Anspruch 5, wobei die zentrale Körperlänge kleiner oder gleich 2,5 mm ist.

7. Der Einsatz nach Anspruch 6, wobei die zentrale Körperlänge kleiner oder gleich 2,0 mm ist.

8. Der Einsatz nach Anspruch 1, wobei sich der verlängerte Teil (236, 242, 244) nur in eine einzige Richtung erstreckt.

9. Der Einsatz nach Anspruch 1, welcher mindestens zwei verlängerte Teile (236, 238, 242, 244, 246) besitzt, welche sich in unterschiedliche Richtungen erstrecken.

10. Der Einsatz nach Anspruch 9, wobei der Einsatz (222) im Wesentlichen ankerförmig ist.

11. Der Einsatz nach Anspruch 9, wobei der Einsatz (240) im Wesentlichen kreuzförmig ist.

12. Ein intracornealer Brechungskorrektur-Einsatz (222, 228, 240, 250) zur Einführung in die Hornhaut eines menschlichen Auges, wobei dieser Einsatz ein physiologisch verträgliches Material umfaßt und zur Implantation innerhalb der menschlichen Hornhaut angepaßt ist und wobei dieser Einsatz weiter einen ersten verlängerten Teil und einen zweiten verlängerten Teil umfaßt, welche sich von diesem in eine andere Richtung erstrecken, wobei einer der ersten und zweiten Teile für den radialen Einsatz in die Hornhaut eines menschlichen Auges gestaltet ist.

13. Der Einsatz nach Anspruch 12, wobei sich vom ersten verlängerten Teil aus der zweite und ein dritter verlängerter Teil erstrecken.

14. Der Einsatz nach Anspruch 12, wobei der Einsatz (222) in Form eines Ankers gestaltet ist.

15. Der Einsatz nach Anspruch 12, wobei der Einsatz (228, 250) in Form eines Boomerangs gestaltet ist.

16. Der Einsatz nach Anspruch 12, wobei der Einsatz (240) in Form eines Kreuzes gestaltet ist.

## Revendications

1. Insert intracornéen (202, 216, 222, 240) pour l'introduction à l'intérieur de la cornée d'un oeil humain, ledit insert comprenant un matériau physiologiquement compatible, et comprenant de plus au moins une partie allongée (236, 242, 244) comportant un composant avec un rayon de courbure, mesuré le long de l'axe centroïdinal de l'insert, supérieur à 5,0 mm, l'insert étant adapté pour l'implantation à l'intérieur d'une cornée humaine avec ledit composant qui s'étend dans une direction méroïdinale, de façon à effectuer ainsi une correction de réfraction.

2. Insert selon la revendication 1, dans lequel ledit rayon de courbure est supérieur à 5,5 mm.

3. Insert selon la revendication 1, dans lequel ledit rayon de courbure est compris entre 6,0 et 9,0 mm.

4. Insert selon la revendication 1, dans lequel ledit rayon de courbure est compris entre 7,0 et 8,0 mm.

5. Insert selon la revendication 1, ayant une longueur centroïdinale inférieure à 3,0 mm.

6. Insert selon la revendication 5, dans lequel ladite longueur centroïdinale est inférieure ou égale à 2,5 mm.

7. Insert selon la revendication 6, dans lequel ladite longueur centroïdinale est inférieure ou égale à 2,0 mm.

8. Insert selon la revendication 1, dans lequel ladite partie allongée (236, 242, 244) ne s'étend que dans une seule direction.

9. Insert selon la revendication 1, comprenant au moins deux parties allongées (236, 238, 242, 244, 246) s'étendant dans des directions différentes.

10. Insert selon la revendication 9, ledit insert (222) étant sensiblement en forme d'ancré.

11. Insert selon la revendication 9, ledit insert (240) étant sensiblement en forme de croix.

12. Insert de correction de réfraction intracornéen (22, 228, 240, 250) pour l'introduction à l'intérieur de la cornée d'un oeil humain, ledit insert comprenant un matériau physiologiquement compatible, et étant adapté pour l'implantation à l'intérieur d'une cornée humaine, ledit insert comprenant de plus une première partie allongée et une deuxième partie allongée s'étendant à partir de celle-ci dans une direction différente, l'une parmi lesdites première et deuxième parties étant configurée pour l'insertion radiale dans la cornée d'un oeil humain.

13. Insert selon la revendication 12, dans lequel ladite première partie allongée comporte ladite deuxième partie et une troisième partie allongée s'étendant à partir de celle-ci.

14. Insert selon la revendication 12, ledit insert (222) étant configuré sous la forme d'une ancre.

15. Insert selon la revendication 12, ledit insert (228, 250) étant configuré sous la forme d'un boomerang.

16. Insert selon la revendication 12, ledit insert (240) étant configuré sous la forme d'une croix.
